# EUROPEAN PATENT SPECIFICATION

(11) **EP 3 347 104 B1**
(45) Date of publication and mention of the grant of the patent: **30.09.2020**
(21) Application number: 16753354.6
(22) Date of filing: 16.08.2016
(51) Int. Cl.: A61K 8/34, A61P 31/04, A01N 39/00, A61Q 19/00, A61K 8/49, A61K 8/368, A61K 31/444, A01N 37/10, A01N 43/16

(54) **LIQUID CONCENTRATE FOR PRESERVING COSMETICS**
FLÜSSIGKONZENTRAT ZUR KONSERVIERUNG VON KOSMETIKA
CONCENTRÉ LIQUIDE POUR LA PRÉSERVATION DE COSMÉTIQUES

(30) Priority: 08.09.2015 DE 102015115024
(43) Date of publication of application: 18.07.2018
(73) Proprietor: Schülke & Mayr GmbH, 22851 Norderstedt (DE)
(72) Inventor: BEILFUSS, Wolfgang, 22339 Hamburg (DE); GRADTKE, Ralf, 25436 Tornesch (DE); SAKULOWSKI, Stefan, 22083 Hamburg (DE); WEBER, Klaus, 20146 Hamburg (DE)
(74) Representative: Grout de Beaufort, François-Xavier
(86) International application number: PCT/EP2016/069429
(87) International publication number: WO 2017/042001

(56) References cited:
- WO-A2-98/20094
- WO-A2-2009/106468
- DE-C1- 3 925 540

## Description

The invention relates to a liquid concentrate which comprises a) one or more aromatic alcohols, b) one or more specific organic acids or salts thereof and c) octenidine dihydrochloride. The liquid concentrate is used for preserving pharmaceutical or cosmetic products. Moreover, the invention relates to a product which comprises the concentrate.

Cosmetic products are often preserved using formulations which comprise organic acids or salts thereof. For example, DE 40 26 765 A discloses a preservative which comprises carboxylic acids or salts thereof, aromatic alcohols and poly(hexamethylene biguanide) salts. The amount of poly(hexamethylene biguanide) salt described in DE 40 26 765 A is 0.1 to 20% by weight. Furthermore, glycerol ether can be present.

Moreover, the product euxyl® K 702 from Schulke & Mayr GmbH (Norderstedt, Federal Republic of Germany) is known which comprises benzoic acid, poly(hexamethylene biguanide) salt, dehydracetic acid and phenoxyethanol. However, the use of cationic surfactants (such as poly(hexamethylene biguanide) salts), especially if they are used in relatively large amounts of, for example, 1% by weight, in anionic-surfactant-based products such as shampoos is problematic since it can result in interaction with the formation of precipitations or effect inactivation. Moreover, according to EU Regulation 944/2013, poly(hexamethylene biguanide) salts have been classed as CMR2 substance since 2015, for which reason they are no longer allowable as constituent in cosmetics.

DE 199 22 538 A1 describes a liquid concentrate which consists of a carboxylic acid component, an alcohol component, a solvent and optionally further auxiliaries, additives and/or active ingredients, wherein the total fraction of components A and B is greater than 45% by weight. According to DE 199 22 538 A1, further active ingredients may be present (although cationic substances are preferably excluded).

The example formulations in DE 199 22 538 A1 comprise at least 20% by weight of organic acid or salt thereof, which is achieved according to the invention of DE 199 22 538 A1 by using the organic acids at least in part in the form of the salts.

Consequently, the present invention was based on the object of providing liquid concentrates for preserving cosmetics which do not automatically comprise a content of organic acid or salt thereof of more than 20% by weight. However, these liquid concentrates should have an adequate preserving effect even when used in a small amount. Moreover, the liquid concentrates should be sufficiently storage-stable even at temperatures of -5°C and in addition should not automatically comprise poly(hexamethylene biguanide) salt.

Surprisingly, it has now been found that this object is achieved by a liquid concentrate according to the invention which comprises
a) one or more aromatic alcohols selected from aryloxyalkanols and arylalkanols, wherein the total amount of component a) is at least 50% by weight, based on the weight of the liquid concentrate,
b) one or more acids selected from benzoic acid, propionic acid, salicylic acid, sorbic acid, 4-hydroxybenzoic acid, dehydracetic acid, formic acid or 10-undecylenic acid, or one or more salts of these acids, wherein the total amount of component b) is at least 8% by weight, stated as free acid or acids and based on the weight of the liquid concentrate, and
c) at least 0.03% by weight of octenidine dihydrochloride, based on the weight of the liquid concentrate.

In a first embodiment, the invention relates to the liquid concentrate. In a second embodiment, the invention relates to the use of the liquid concentrate for preserving pharmaceutical (in particular dermatological) and cosmetic products. In a third embodiment, the invention relates to products which comprise the liquid concentrate.

Concentrates according to the invention are characterized in that the amount of component b), i.e. organic acid or salt thereof, required for efficacy is not necessarily high, with component c) octenidine dihydrochloride (which is not present as an antimicrobial active ingredient) surprisingly assisting the antimicrobial efficacy of components a) and b). Liquid concentrates according to the invention, moreover, are storage-stable (even at a low storage temperature of for example 5°C) and are adequately effective in typical amounts suitable for preserving cosmetics.

### a) Aromatic alcohol

Liquid concentrates according to the invention comprise a) one or more aromatic alcohols selected from aryloxyalkanols and arylalkanols, wherein the total amount of component a) is at least 50% by weight, based on the weight of the liquid concentrate. Examples of aromatic alcohols are benzyl alcohol, phenoxyethanol, phenethyl alcohol, 3-phenylpropanol and phenoxypropanol or mixtures thereof.

Aryloxyalkanols used according to the invention preferably have the formula ArO(CHR)ₙOH where R = independently H (for n ≥ 2) or C₁ to C₆ alkyl, where n is an integer and preferably 2 to 10, more preferably 2 to 6 and in particular 2 or 3. While the group Ar can be a ring-substituted or unsubstituted aryl group, preference is given to unsubstituted aryl, e.g. phenyl or naphthyl. Examples of aryloxyalkanols used according to the invention are phenoxyethanol and phenoxypropanols. Preferred phenoxypropnaols are 1-phenoxypropanol-2, 2-phenoxypropanol-1 or mixtures thereof, and 3-phenoxypropanol-1.

Arylalkanols used according to the invention have the formula Ar(CHR)ₙOH where R = independently H or C₁ to C₆ alkyl, where n is an integer and preferably 1 to 10, more preferably 1 to 6 and in particular 1, 2, 3 or 4. While the group Ar can be a ring-substituted or unsubstituted aryl group, preference is given to unsubstituted aryl, e.g. phenyl or naphthyl. Examples of arylalkanols are 3-phenylpropanol-1, phenethyl alcohol, veratryl alcohol (3,4-dimethoxyphenylmethyl alcohol), benzyl alcohol and 2-methyl-1-phenyl-2-propanol, preferably phenethyl alcohol or benzyl alcohol.

A preferred alcohol component is a1) phenoxyethanol. Alternatively, component a) is preferably specifically a2) benzyl alcohol. Further alternatively, component a) is preferably a3) 3-phenylpropanol. Furthermore alternatively, component a) is preferably a4) phenethyl alcohol.

Preferably, the total amount of component a), i.e. the total amount of the aromatic alcohols selected form aryloxyalkanols and arylalkanols, is 55 to 90% by weight, preferably 60 to 85% by weight, in particular 65 to 80% by weight, such as 70 to 77% by weight, for example about 74% by weight, in each case based on the weight of the liquid concentrate.

### b) Organic acid or salt thereof

Moreover, the liquid concentrate according to the invention comprises one or more organic acids selected from benzoic acid, propionic acid, salicylic acid, sorbic acid, 4-hydroxybenzoic acid, dehydracetic acid, formic acid or 10-undecylenic acid, or one or more salts of these acids, wherein the total amount of component b) is at least 8% by weight, stated as free acid or acids and based on the weight of the liquid concentrate. Examples of these acids are benzoic acid, sorbic acid, dehydracetic acid, salicylic acid, 10-undecylenic acid and salts thereof, and mixtures thereof. Preferably, the acid is selected from benzoic acid, propionic acid, salicylic acid, sorbic acid, 4-hydroxybenzoic acid and dehydracetic acid. Particularly preferred organic acids are benzoic acid, dehydracetic acid and sorbic acid.

In a very preferred embodiment, component b) is specifically b1) benzoic acid, b2) dehydracetic acid or b3) a combination of benzoic acid and dehydracetic acid.

Alternatively, component b) is preferably a combination of carboxylic acid(s) and carboxylic acid salt(s), a combination of benzoic acid and sodium benzoate, for example, being preferred as component b).

Preferably, the total amount of component b), i.e. the total amount of the specified organic acids and salts thereof, is 10 to 30% by weight, preferably 12 to 27% by weight, in particular 14 to 25% by weight, such as 16 to 22% by weight, in each case stated as free acid or acids and based on the weight of the liquid concentrate.

### c) Octenidine dihydrochloride

Furthermore, the liquid concentrate according to the invention comprises at least 0.03% by weight of octenidine dihydrochloride, based on the weight of the liquid concentrate. A preferred minimum amount of c) octenidine dihydrochloride is 0.04% by weight, in particular 0.05% by weight.

According to the invention, octenidine dihydrochloride is not used as an antimicrobial active ingredient, but instead as a cationic surfactant, and specifically for improving the interfacial activity (when used in the end product). The quaternary structure of octenidine dihydrochloride is pH-dependent. Octenidine dihydrochloride can be in the form of the bispyridinium salt, or in the form of N,N'- (1,10-decanediyldi-1(4H)-pyridinyl-4-ylidene)bis-1-octaneamine. Furthermore, the term octenidine dihydrochloride encompasses the various prototropes of the compound, as disclosed for example in DE 196 47 692 A1.

According to the invention, particular preference is given to an octenidine dihydrochloride grade which is produced in aqueous solution (compare the teaching of DE 100 05 853 A) and which is consequently free from undesired organic solvents such as, for example, dimethylformamide.

Preferably, the amount of octenidine dihydrochloride in the liquid concentrate according to the invention is in the range from 0.04 to 0.2% by weight, more preferably 0.05 to 0.1% by weight, such as about 0.05% by weight, in each case based on the weight of the liquid concentrate.

### d) Glycerol monoalkyl ethers

The liquid concentrate according to the invention furthermore preferably comprises d) one or more 1- or 2-(C₃- to C₂₄-alkyl) glycerol ethers (glycerol monoalkyl ethers). A content of d) glycerol monoalkyl ethers improves (i.e. reduces) the surface tension of aqueous dilutions of the liquid concentrate according to the invention, and thus when using the liquid concentrates according to the invention as preservatives improves the wetting as a result of these dilutions and thereby assists their antimicrobial effect.

Examples of d) glycerol monoalkyl ethers optionally used according to the invention are glycerol monoalkyl ethers substituted with saturated or unsaturated, branched or branched alkyl in one or two position (i.e. symmetrical or asymmetrical), such as dodecyl glycerol ether, decyl glycerol ether, octyl glycerol ether, propyl glycerol ether, octadecyl glycerol ether (batyl alcohol), hexadecyl glycerol ether (chimyl alcohol) and octadenyl glycerol ether (selachyl alcohol). Preference is given to 1-monoalkyl glycerol ethers with saturated (branched or unbranched) C₃ to C₁₈-alkyl, particularly preferably saturated and branched C₆ to C₁₂-alkyl. Very particular preference is given to 1-(2-ethylhexyl) glycerol ether (ethylhexylglycerol, Sensiva® SC 50 from Schülke & Mayr GmbH, Norderstedt, Federal Republic of Germany).

Preferably, the total amount of component d) is 0.5 to 10% by weight, preferably 1.0 to 5% by weight, in particular 1.5 to 2.5% by weight, such as about 2% by weight, in each case based on the weight of the liquid concentrate.

Ethylhexylglycerol serves an amphiphilic component for improving the interfacial activity (in the end product). The combination of octenidine dihydrochloride with ethylhexylglycerol is particularly advantageous for improving the interfacial activity and for boosting the microbicidal efficacy of the active ingredient components (i.e. a) aromatic alcohol and b) acid).

The combination of octendine dihydrochloride with the optionally used ethylhexylglycerol leads to a surprisingly strong interaction of the components, which manifests itself in a change of physical-chemical properties, e.g. improved solubility in water of ethylhexylglyercol and octenidine dihydrochloride and lower interfacial activity. A comparably strong interaction was not observed between polybiguanide and ethylhexylglycerol.

The combination of glycerol ethers and octenidine dihydrochloride has a low (dynamic) interfacial tension, the same is true for the combination of aromatic alcohol and octenidine dihydrochloride or aromatic alcohol and glycerol ethers, in particular for the combination of aromatic alcohol and glycerol ethers and octenidine dihydrochloride. The low interfacial tension makes a (considerable) contribution to the antimicrobial efficacy, to the wetting of substrates such as particles, polymers, fibres, microorganisms and biofilms. For the machine loading of wipes with product preserved according to the invention (see below), a low dynamic interfacial tension is advantageous.

The antimicrobial active ingredients (alcohol component and acid component) are advantageously assisted in their microbicidal efficacy by the interface-active additives such as cationic octenidine dihydrochloride and amphiphilic glycerol ethers. In particular, the combination of octenidine dihydrochloride and 2-ethylhexylglycerol leads to an enhancement of the microbicidal efficacy of the active ingredient components.

### e) Antioxidant

Furthermore, preference is given to concentrates according to the invention which comprise e) one or more antioxidants.

Antioxidants that are optionally used according to the present invention are preferably selected from the group consisting of 3-tert-butyl-4-hydroxyanisole, 2,6-di-tert-butyl-p-cresol, tocopherol, in particular vitamin E, and its derivatives, in particular vitamin E derivatives such as vitamin E acetate, vitamin E linoleate, vitamin E nicotinate and vitamin E succinate, p-hydroxybenzoic acid esters, in particular its methyl, ethyl, propyl or butyl esters, dimethyloldimethylhydantoin and N-acylamino acids and salts thereof, in particular N-octanoylglycine. In particular, the antioxidants are selected from the group consisting of vitamin E and its derivatives, 3-tert-butyl-4-hydroxyanisole and 2,6-di-tert-butyl-p-cresol, with vitamin E being most preferred.

The tocopherols are particularly desirable antioxidants with regard to the applications associated with strictly imposed regulations and toxicity tests of the concentrates according to the invention during the manufacture of cosmetics and pharmaceuticals.

Preferred antioxidants are selected from 2,6-di-tert-butyl-p-cresol (BHT), 3-tert-butyl-4-hydroxyanisole (BHA), vitamin E and its derivatives. A very particularly preferred component e) is vitamin E.

Preferred amounts of the optional component e), i.e. total amounts of the one or more antioxidants, are 1 to 1000 ppm, preferably 5 to 500 ppm, such as about 10 or about 200 ppm (weight/weight of the liquid concentrate).

Preferred amounts of the optional component e), i.e. total amounts of the one or more antioxidants, are - when using a) aryloxyalkanols - 1 to 100 ppm, preferably 5 to 50 ppm, such as about 10 ppm (weight/weight of the liquid concentrate).

If one or more arylalkanols, e.g. benzyl alcohol or phenethyl alcohol, are used as aromatic alcohol, a preferred total concentration of component e) is 1 to 1000 ppm, such as 50 to 500 ppm, for example about 200 ppm, of antioxidant, e.g. vitamin E as component e) (in each case weight/weight of the liquid concentrate).

### Use

Liquid concentrates according to the invention are used for preserving pharmaceutical (in particular dermatological) or cosmetic products, such as e.g. for preserving shampoos, hand washing lotions and also shower and foam baths, and in particular also for preserving wet wipe emulsions.

### Product

In a further embodiment, the present invention relates to a product, for example a pharmaceutical (primarily dermatological) or cosmetic product which comprises the liquid concentrate. Typically, the liquid concentrate is used in an amount of from 0.1 to 2% by weight, based on the weight of the preserved product, preferably 0.3 to 1.5% by weight, more preferably 0.5 to 1.0% by weight, such as about in an amount of 0.75% by weight.

Liquid concentrates according to the invention thus offer the following advantages:
- very good compatibility and efficacy in conjunction with cationic and nonionic systems,
- good compatibility and efficacy in anionic systems,
- good wetting of hard and flexible surfaces, in particular good wetting of wipe materials,
- the concentrate is low-water and low in chloride ions.

The liquid concentrate:
- comprises no polymeric fractions,
- is advantageous on account of using monosubstances,
- is free from polyaminopropyl biguanide,
- is cost-effective,
- comprises defined ingredients of high purity and grade,
- produces a multifunctional efficacy,
- exhibits a booster effect,
- does not automatically comprise polymeric fractions (such as poly(hexyamethylene biguanide) salts) as active ingredients and
- does not automatically comprise water in order to bring the components prescribed according to the invention into solution (alternatively, a limited amount of water may be present),
   the concentrate can consequently be readily stored.

### Examples

### Method of determining the preserving effect of chemical preservatives in cosmetic formulations (Koko test)

The test described below serves to assess the preserving effect of chemical preservatives in cosmetic formulations.

### Principle

With the help of the described method, the aim is to test the efficacy of chemical preservatives with regard to pack preservation for cosmetic formulations. For this purpose, in different experimental batches to the nonpreserved samples, the preservatives to be investigated are added in different concentrations. A continual germ loading is achieved through periodic inoculation of the experimental batches. In parallel to the inoculation, smears of the individual batches are performed in each case directly beforehand. An assessment is made with regard to the microbial growth of the smears. The longer the period until the first appearance of microbial growth, the more effective a preservative.

### Procedure

In each case, 25 g of the cosmetic to be tested is weighed into wide-neck bottles with screw closure (LDPE). The preservatives to be investigated are added to in each case separate batches in their use concentrations. (Samples which have been sent already preserved for the investigation receive no further biocide addition.) A nonpreserved sample serves in each case as growth control. Two days after adding the preservatives, the samples are infected with 0.1 ml of an inoculation solution consisting of the test organisms listed below. The inoculation solution has a titer of about 10⁸- 10⁹ germs/ml.

| | | | | |
|---|---|---|---|---|
| **Bacteria** | Gram positive | | *Staphylococcus aureus* | **ATCC 6538** |
| | | | *Kocuria rhizophila* | **ATCC 9341** |
| | Gram negative | Entero-bacteria | *Enterobacter gergoviae* | **ATCC 33028** |
| | | | *Escherichia coli* | **ATCC 11229** |
| | | | *Klebsiella pneumoniae* | **ATCC 4352** |
| | | Pseudo-monads | *Pseudomonas aeruginosa* | **ATCC 9027** |
| | | | *pseudomonas fluorescens* | **ATCC 17397** |
| | | | *Pseudomonas putida* | **ATCC 12633** |
| **Yeast** | | | *Candida albicans* | **ATCC 10231** |
| **Moulds** | | | *Aspergillus brasiliensis* | **ATCC 16404** |
| | | | *Penicillium pinophilum* | **ATCC 36839** |

The test batches are then inoculated once a week and smeared on agar plates once a week (casein peptone-soya flour peptone agar (CSA) for bacteria and sabourand-dextrose-agar (SA) for yeasts and moulds), with the first smear (sterility test) taking place both on inhibited (TLSH) as well as on noninhibited culture media in order to discover as far as possible all starting contaminations. Assessment of the microbial growth of the smears is carried out after incubation for three days at 25°C. To be on the safe side, negative smears are observed for a further two days and assessed again. Assessment of the preserving effect of the individual product concentrations is performed in a semi-quantitative method via the growth of the individual smears.

| | | | | | |
|---|---|---|---|---|---|
| - | = | growth-free | ++ | = | moderate growth |
| + | = | weak growth | +++ | = | strong growth |

The growth is differentiated according to bacteria, yeasts and moulds ("B" = bacteria, "Y" = yeasts, "M" = moulds, "Sp" = spore-forming bacteria, "/" = the test was terminated). The experiment is carried out for a maximum of six weeks, i.e. over six inoculation cycles, or terminated after strong growth (+++) multiple times.

### Assessment of the results

The sample is well preserved if, under the aforementioned laboratory conditions, it withstands a period of six weeks without microbial attack of the sample batches, i.e. microbial growth cannot be detected even after the sixth inoculation. From experience with this test method over a year, it is possible to deduce a microbiological stability recommended for cosmetics of more than 30 months.

### Tested concentrates

The following concentrates were tested (quantitative data in % by weight):

| **Constituent** | **A*** | **B*** | **C*** | **D** | **E*** |
|---|---|---|---|---|---|
| Phenoxyethanol | 73.90 | 73.90 | 73.90 | 73.90 | - |
| Benzoic acid | 12.00 | 12.00 | 12.00 | 12.00 | - |
| Polybiguanide (20% strength) | 5.00 | - | - | - | - |
| Dehydracetic acid | 7.00 | 7.00 | 7.00 | 7.00 | - |
| Demineralized water | - | 5.00 | 4.99 | 4.95 | 99.95 |
| Octenidine dihydrochloride | - | - | 0.01 | 0.05 | 0.05 |
| 1-(2-Ethylhexyl) glycerol ether | 2.00 | 2.00 | 2.00 | 2.00 | - |
| Sodium hydroxide (45% strength) | 0.10 | 0.10 | 0.10 | 0.10 | - |

| | | | | | |
|---|---|---|---|---|---|
| *Comparison concentrate | | | | | |

| **Test material** | | **Sterility control** | **Inoculation cycles** | | | | | |
|---|---|---|---|---|---|---|---|---|
| | | | 1 | 2 | 3 | 4 | 5 | 6 |
| 1 | PEG-free Emulsion for baby wipes | - | +++ B.S.H | +++ B.S.H | / | | | |
| 2 | + 0,50% A | - | - | + S | ++ S | ++ S | ++ S | ++ S |
| 3 | + 0,75% A | - | - | - | - | - | - | + S |
| 4 | + 1,00% A | - | - | - | - | - | - | - |
| 5 | + 0,50% B | - | ++ S | +++ S | +++ S | / | | |
| 6 | + 0,75% B | - | + S | + S | + S | + S | - | + S |
| 7 | + 1,00% B | | | | | | | |
| 8 | + 0,50% C | - | ++ S | +++ S | +++ S | / | | |
| 9 | + 0,75% C | - | S | + S | - | - | + S | + S |
| 10 | + 1,00% C | | | | | | | |
| 11 | + 0,50% D | - | +++ S | +++ S | / | | | |
| 12 | + 0,75% D | | | | | | | |
| 13 | + 1,00% D | | | | | | | |
| 14 | + 1,00% E | - | +++ B.S.H | +++ B.S.H | / | | | |

As these tests reveal, a polyethylene glycol-free emulsion for baby wipes without preservative is inadequately antimicrobially equipped (cf. Test 1). A standard commercial concentrate (compare concentrate A) antimicrobially equips this emulsion in a use concentration of 0.75% (test results 2 to 4). Moreover, as test results 5 to 7 reveal, a formulation which corresponds to concentrate A but comprises no polyhexamethylene biguanide, in a use concentration of 0.75% does not adequately antimicrobially equip the emulsion.

A formulation with only 0.01% by weight octenidine dihydrochloride (concentrate C), in a use concentration of 0.75%, also has an inadequate preserving action (test results 8 to 10). By contrast, a liquid concentrate according to the invention (concentrate D) - in a use concentration of 0.75% - antimicrobially equips the emulsion adequately (test results 14 to 16), with the improved effect in the presence of more than 0.01% by weight of octenidine dihydrochloride in the concentrate (see the comparison concentrates B and C and, by contrast, concentrate D according to the invention) therefore being surprising because 0.05% of octenidine dihydrochloride on its own does not have an antimicrobial effect (see concentrate E), not even in a use concentration of 1.0% by weight in the emulsion equipped therewith (see test result 14).

Octenidine dihydrochloride thus assists the antimicrobial efficacy although it does not have an antimicrobial effect on its own in the stated use concentration.

## Claims

1. Liquid concentrate which comprises
a) one or more aromatic alcohols selected from aryloxyalkanols and arylalkanols, wherein the total amount of component a) is at least 50% by weight, based on the weight of the liquid concentrate,
b) one or more acids selected from benzoic acid, propionic acid, salicylic acid, sorbic acid, 4-hydroxybenzoic acid, dehydracetic acid, formic acid or 10-undecylenic acid, or one or more salts of these acids, wherein the total amount of component b) is at least 8% by weight, stated as free acid or acids and based on the weight of the liquid concentrate, and
c) at least 0.03% by weight of octenidine dihydrochloride, based on the weight of the liquid concentrate.

2. Liquid concentrate according to Claim 1, **characterized in that** the aryloxyalkanol is selected from phenoxyethanol or phenoxypropanol, preferably phenoxyethanol.

3. Liquid concentrate according to Claim 1 or 2, **characterized in that** the arylalkanol is 3-phenypropanol-1, phenethyl alcohol, veratryl alcohol, benzyl alcohol or 2-methyl-1-phenyl-2-propanol, preferably phenethyl alcohol or benzyl alcohol.

4. Liquid concentrate according to one of the preceding claims, **characterized in that** the total amount of component a) is 55 to 90% by weight, preferably 60 to 85% by weight, in particular 65 to 80% by weight, such as 70 to 77% by weight, for example about 74% by weight, in each case based on the weight of the liquid concentrate.

5. Liquid concentrate according to one of the preceding claims, **characterized in that** the acid is selected from benzoic acid, propionic acid, salicylic acid, sorbic acid, 4-hydroxybenzoic acid and dehydracetic acid,
wherein component b) is preferably selected from benzoic acid, dehydracetic acid and mixtures thereof.

6. Liquid concentrate according to one of the preceding claims, **characterized in that** the total amount of component b) is 10 to 30% by weight, preferably 12 to 27% by weight, in particular 14 to 25% by weight, such as 16 to 22% by weight, in each case stated as free acid or acids and based on the weight of the liquid concentrate.

7. Liquid concentrate according to one of the preceding claims, **characterized in that** the amount of octenidine dihydrochloride c) is 0.04 to 0.2% by weight, preferably 0.05 to 0.1% by weight, in each case based on the weight of the liquid concentrate.

8. Liquid concentrate according to one of the preceding claims, **characterized in that** it also comprises
d) one or more 1- or 2-(C₃ to C₂₄-alkyl) glycerol ethers.

9. Liquid concentrate according to Claim 8, **characterized in that** the amount of component d) is 0.5 to 10% by weight, preferably 1.0 to 5% by weight, in particular 1.5 to 2.5% by weight, such as about 2% by weight, in each case based on the weight of the liquid concentrate.

10. Liquid concentrate according to one of the preceding claims, **characterized in that** it also comprises
e) one or more antioxidants.

11. Liquid concentrate according to Claim 10, **characterized in that** the antioxidant is selected from 2,6-di-tert-butyl-p-cresol (BHT), 3-tert-butyl-4-hydroxyanisole (BHA) and vitamin E and its derivatives, wherein component e) is preferably vitamin E.

12. Liquid concentrate according to Claim 10 or 11, **characterized in that** the amount of component e) is 1 to 1000 ppm, preferably 5 to 500 ppm, such as about 10 or about 200 ppm (weight/weight of the liquid concentrate).

13. Use of the liquid concentrate according to one of the preceding claims for preserving pharmaceutical (in particular dermatological) or cosmetic products.

14. Product which comprises the liquid concentrate according to one of Claims 1 to 12.

15. Product according to Claim 14, **characterized in that** it is a pharmaceutical (preferably dermatological) or cosmetic product.

## Patentansprüche

1. Flüssigkonzentrat, das umfasst
a) einen oder mehrere aromatische Alkohole, ausgewählt aus Aryloxyalkanolen und Arylalkanolen, wobei die Gesamtmenge von Komponente a) bezogen auf das Gewicht des Flüssigkonzentrats mindestens 50 Gewichts-% beträgt,
b) eine oder mehrere Säuren, ausgewählt aus Benzoesäure, Propionsäure, Salicylsäure, Sorbinsäure, 4-Hydroxybenzoesäure, Dehydracetsäure, Ameisensäure oder 10-Undecylensäure, oder ein oder mehrere Salze dieser Säuren, wobei die Gesamtmenge von Komponente b) mindestens 8 Gewichts-% beträgt, angegeben als freie Säure oder Säuren und bezogen auf das Gewicht des Flüssigkonzentrats, und
c) bezogen auf das Gewicht des Flüssigkonzentrats mindestens 0,03 Gewichts-% Octenidindihydrochlorid.

2. Flüssigkonzentrat nach Anspruch 1, **dadurch gekennzeichnet, dass** das Aryloxyalkanol ausgewählt ist aus Phenoxyethanol oder Phenoxypropanol, vorzugsweise Phenoxyethanol.

3. Flüssigkonzentrat nach Anspruch 1 oder 2, **dadurch gekennzeichnet, dass** das Arylalkanol 3-Phenylpropanol-1, Phenethylalkohol, Veratrylalkohol, Benzylalkohol oder 2-Methyl-1-phenyl-2-propanol ist, vorzugsweise Phenethylalkohol oder Benzylalkohol.

4. Flüssigkonzentrat nach einem der vorstehenden Ansprüche, **dadurch gekennzeichnet, dass** die Gesamtmenge von Komponente a) 55 bis 90 Gewichts-% beträgt, vorzugsweise 60 bis 85 Gewichts-%, insbesondere 65 bis 80 Gewichts-%, wie 70 bis 77 Gewichts-%, beispielsweise etwa 74 Gewichts-%, jeweils bezogen auf das Gewicht des Flüssigkonzentrats.

5. Flüssigkonzentrat nach einem der vorstehenden Ansprüche, **dadurch gekennzeichnet, dass** die Säure ausgewählt ist aus Benzoesäure, Propionsäure, Salicylsäure, Sorbinsäure, 4-Hydroxybenzoesäure und Dehydracetsäure,
wobei Komponente b) vorzugsweise ausgewählt ist aus Benzoesäure, Dehydracetsäure und Mischungen davon.

6. Flüssigkonzentrat nach einem der vorstehenden Ansprüche, **dadurch gekennzeichnet, dass** die Gesamtmenge von Komponente b) 10 bis 30 Gewichts-% beträgt, vorzugsweise 12 bis 27 Gewichts-%, insbesondere 14 bis 25 Gewichts-%, wie 16 bis 22 Gewichts-%, jeweils angegeben als freie Säure oder Säuren und bezogen auf das Gewicht des Flüssigkonzentrats.

7. Flüssigkonzentrat nach einem der vorstehenden Ansprüche, **dadurch gekennzeichnet, dass** die Menge an Octenidindihydrochlorid c) 0,04 bis 0,2 Gewichts-% beträgt, vorzugsweise 0,05 bis 0,1 Gewichts-%, jeweils bezogen auf das Gewicht des Flüssigkonzentrats.

8. Flüssigkonzentrat nach einem der vorstehenden Ansprüche, **dadurch gekennzeichnet, dass** es ferner
d) einen oder mehrere 1- oder 2-(C₃- bis C₂₄-Alkyl)glycerinether umfasst.

9. Flüssigkonzentrat nach Anspruch 8, **dadurch gekennzeichnet, dass** die Menge von Komponente d) 0,5 bis 10 Gewichts-% beträgt, vorzugsweise 1,0 bis 5 Gewichts-%, insbesondere 1,5 bis 2,5 Gewichts-%, wie etwa 2 Gewichts-%, jeweils bezogen auf das Gewicht des Flüssigkonzentrats.

10. Flüssigkonzentrat nach einem der vorstehenden Ansprüche, **dadurch gekennzeichnet, dass** es ferner
e) ein oder mehrere Antioxidantien umfasst.

11. Flüssigkonzentrat nach Anspruch 10, **dadurch gekennzeichnet, dass** das Antioxidans ausgewählt ist aus 2,6-Di-tert-butyl-p-kresol (BHT), 3-tert-Butyl-4-hydroxyanisol (BHA) und Vitamin E und seinen Derivaten, wobei Komponente e) vorzugsweise Vitamin E ist.

12. Flüssigkonzentrat nach Anspruch 10 oder 11, **dadurch gekennzeichnet, dass** die Menge von Komponente e) 1 bis 1000 ppm beträgt, vorzugsweise 5 bis 500 ppm, wie etwa 10 oder etwa 200 ppm (Gewicht/Gewicht des Flüssigkonzentrats).

13. Verwendung des Flüssigkonzentrats nach einem der vorstehenden Ansprüche zum Konservieren pharmazeutischer (insbesondere dermatologischer) oder kosmetischer Produkte.

14. Produkt, das das Flüssigkonzentrat nach einem der Ansprüche 1 bis 12 umfasst.

15. Produkt nach Anspruch 14, **dadurch gekennzeichnet, dass** es ein pharmazeutisches (vorzugsweise dermatologisches) oder kosmetisches Produkt ist.

## Revendications

1. Concentré liquide qui comprend
a) un ou plusieurs alcools aromatiques sélectionnés parmi les aryloxyalkanols et les arylalkanols, dans lequel la quantité totale du composant a) est d'au moins 50 % en poids, sur la base du poids du concentré liquide,
b) un ou plusieurs acides sélectionnés parmi l'acide benzoïque, l'acide proprionique, l'acide salicylique, l'acide sorbique, l'acide 4-hydroxybenzoïque, l'acide déhydracétique, l'acide formique ou l'acide 10-undécylénique, ou un ou plusieurs sels de ces acides, dans lequel la quantité totale du composant b) est d'au moins 8 % en poids, indiqué en tant qu'acide ou acides libres et sur la base du poids du concentré liquide, et
c) au moins 0,03 % en poids de dihydrochlorure d'octénidine, sur la base du poids du concentré liquide.

2. Concentré liquide selon la revendication 1, **caractérisé en ce que** l'aryloxyalkanol est sélectionné parmi le phénoxyéthanol ou le phénoxypropanol, de préférence le phénoxyéthanol.

3. Concentré liquide selon la revendication 1 ou 2, **caractérisé en ce que** l'arylalkanol est du 3-phénylpropanol-1, de l'alcool de phénéthyle, de l'alcool de vératryle, de l'alcool de benzyle ou du 2-méthyl-1-phényl-2-propanol, de préférence de l'alcool de phénéthyle ou de l'alcool de benzyle.

4. Concentré liquide selon l'une des revendications précédentes, **caractérisé en ce que** la quantité totale du composant a) est de 55 à 90 % en poids, de préférence de 60 à 85 % en poids, en particulier de 65 à 80 % en poids, telle que de 70 à 77 % en poids, par exemple d'environ 74 % en poids, dans chaque cas sur la base du poids du concentré liquide.

5. Concentré liquide selon l'une des revendications précédentes, **caractérisé en ce que** l'acide est sélectionné parmi l'acide benzoïque, l'acide propionique, l'acide salicylique, l'acide sorbique, l'acide 4-hydroxybenzoïque et l'acide déhydracétique,
dans lequel le composant b) est de préférence sélectionné parmi l'acide benzoïque, l'acide déhydracétique et des mélanges de ceux-ci.

6. Concentré liquide selon l'une des revendications précédentes, **caractérisé en ce que** la quantité totale du composant b) est de 10 à 30 % en poids, de préférence de 12 à 27 % en poids, en particulier de 14 à 25 % en poids, telle que de 16 à 22 % en poids, dans chaque cas indiqué en tant qu'acide ou acides libres et sur la base du poids du concentré liquide.

7. Concentré liquide selon l'une des revendications précédentes, **caractérisé en ce que** la quantité de dihydrochlorure d'octénidine c) est de 0,04 à 0,2 % en poids, de préférence de 0,05 à 0,1 % en poids, dans chaque cas sur la base du poids du concentré liquide.

8. Concentré liquide selon l'une des revendications précédentes, **caractérisé en ce qu'**il comprend également
d) un ou plusieurs éthers de 1- ou 2-(C₃ à C₂₄-alkyle) glycérol.

9. Concentré liquide selon la revendication 8, **caractérisé en ce que** la quantité du composant d) est de 0,5 à 10 % en poids, de préférence de 1,0 à 5 % en poids, en particulier de 1,5 à 2,5 % en poids, telle qu'environ 2 % en poids, dans chaque cas sur la base du poids du concentré liquide.

10. Concentré liquide selon l'une des revendications précédentes, **caractérisé en ce qu'**il comprend également
e) un ou plusieurs antioxydants.

11. Concentré liquide selon la revendication 10, **caractérisé en ce que** l'antioxydant est sélectionné parmi le 2,6-di-tert-butyl-p-crésol (BHT), le 3-tert-butyl-4-hydroxyanisole (BHA) et la vitamine E et ses dérivés, dans lequel le composant e) est de préférence la vitamine E.

12. Concentré liquide selon la revendication 10 ou 11, **caractérisé en ce que** la quantité du composant e) est de 1 à 1 000 ppm, de préférence de 5 à 500 ppm, telle qu'environ de 10 ou environ de 200 ppm (poids/poids du concentré liquide).

13. Utilisation du concentré liquide selon l'une des revendications précédentes pour conserver des produits pharmaceutiques (en particulier dermatologiques) ou cosmétiques.

14. Produit qui comprend le concentré liquide selon l'une des revendications 1 à 12.

15. Produit selon la revendication 14, **caractérisé en ce qu'il** s'agit d'un produit pharmaceutique (de préférence dermatologique) ou cosmétique.
